# EUROPÄISCHE PATENTSCHRIFT

(11) **EP 1 112 243 B1**
(45) Veröffentlichungstag und Bekanntmachung des Hinweises auf die Patenterteilung: **03.03.2004**
(21) Anmeldenummer: 99968663.7
(22) Anmeldetag: 06.09.1999
(51) Int. Cl.: C07C 43/13, C07C 41/03, C08G 65/26, C07C 67/31

(54) **VERFAHREN ZUR HERSTELLUNG VON FETTALKOHOLALKOXYLATEN**
METHOD FOR THE PREPARATION OF FATTY ALCOHOL ALKOXYLATES
PROCEDE DE PRODUCTION D'ALCOXYLATS D'ALCOOLS GRAS

(30) Priorität: 07.09.1998 DE 19840846
(43) Veröffentlichungstag der Anmeldung: 04.07.2001
(73) Patentinhaber: BASF AKTIENGESELLSCHAFT, 67056 Ludwigshafen (DE)
(72) Erfinder: GROSCH, Georg, Heinrich, D-67098 Bad Dürkheim (DE); LARBIG, Harald, D-67063 Ludwigshafen (DE); LORENZ, Reinhard, D-67117 Limburgerhof (DE); JUNGE, Dieter, D-67227 Frankenthal (DE); OPPENLÄNDER, Knut, D-67061 Ludwigshafen (DE); GÜNTHER, Wolfgang, D-67582 Mettenheim (DE); AUS DEM KAHMEN, Martin, D-67071 Ludwigshafen (DE); MOHR, Jürgen, D-67269 Grünstadt (DE); HARRE, Kathrin, D-01109 Dresden (DE)
(74) Vertreter: Isenbruck, Günter
(86) Internationale Anmeldenummer: PCT/EP1999/006529
(87) Internationale Veröffentlichungsnummer: WO 2000/014045

(56) Entgegenhaltungen:
- EP-A- 0 659 798
- US-A- 5 158 922
- DATABASE WPI Section Ch, Week 9411 Derwent Publications Ltd., London, GB; Class A25, AN 94-089387 XP002104684 & JP 06 041293 A (ASAHI GLASS), 15. Februar 1994 (1994-02-15)
- DATABASE WPI Section Ch, Week 9440 Derwent Publications Ltd., London, GB; Class A25, AN 94-322252 XP002104683 & JP 06 248068 A (ASAHI GLASS), 6. September 1994 (1994-09-06)
- DATABASE WPI Section Ch, Week 9202 Derwent Publications Ltd., London, GB; Class A25, AN 1992-014257 XP002125052 & JP 03 265627 A (ASAHI GLASS), 26. November 1991 (1991-11-26)

## Beschreibung

Alkoxylate von einwertigen aliphatischen Alkoholen mit 6 bis 24 C-Atomen, auch als Fettalkoholpolyalkylenglykolether bezeichnet, sind bedeutende organische Spezialchemikalien und werden beispielsweise als Tenside oder Kraftstoffadditive eingesetzt. Ihre Herstellung erfolgt durch katalytische ringöffnende Polymerisation von Alkylenoxiden, wie Ethylenoxid, Propylenoxid oder Butylenoxid, wobei als Initiator mit aktivem Wasserstoff der entsprechende C₆-C₂₄-Alkohol eingesetzt wird. Als Katalysatoren werden üblicherweise basische Metallsalze, Metalloxide und - hydroxyde eingesetzt.

Große Bedeutung als Katalysator hat bisher Kaliumhydroxyd, das jedoch nur eine mäßige Aktivität aufweist. Ferner ist die Aufarbeitung der gebildeten Fettalkoholalkoxylate bei Verwendung von Kaliumhydroxyd als Katalysator aufwendig, da letzteres mit Säure neutralisiert und durch Kristallisation abgetrennt werden muß. Bei der Alkoxylierung von Hydroxysäureestern, beispielsweise des Glycerins, kommt es in Gegenwart von KOH zur Esterspaltung.

Als heterogene Katalysatoren werden auch schwerlösliche basische Oxide oder Hydroxyde, wie Magnesiumoxid oder Hydrotalcit, eingesetzt. DE-A 41 15 149 beschreibt die Verwendung von Hydrotalcit als Katalysator zur Herstellung von Fettalkoholpolyalkylenglykolethern. Die katalytische Aktivität der schwerlöslichen basischen Oxide oder Hydroxide ist jedoch oft nur mäßig und übertrifft die des Kaliumhydroxids nur selten. Auch ist die Abtrennung der Katalysatoren von den gebildeten Fettalkoholalkoxylaten oft langwierig und gelingt nur unter Zusatz von Hilfsstoffen.

EP-A 0 659 798 offenbart die Verwendung von geträgerten Doppelmetallcyanid-Komplexkatalysatoren, wobei die Doppelmetallcyamid-Verbindung in einen Polyurethanschaum als Träger eingeschäumt ist, als Epoxid-Polymerisationskatalysatoren.

Aufgabe der Erfindung ist es, einen heterogenen Katalysator für die Herstellung von Fettalkoholalkoxylaten durch Alkoxylierung von einwertigen aliphatischen Alkoholen mit 6 bis 24 C-Atomen bereitzustellen, der eine hohe katalytische Aktivität aufweist und sich in einfacher Weise vom gebildeten Fettallcoholalkoxylat abtrennen läßt.

Die Lösung der Aufgabe geht aus von dem Verfahren zur Herstellung von Fettalkoholalkoxylaten durch Alkoxylierung von einwertigen aliphatischen Alkoholen mit 6 bis 24 C-Atomen oder von Estern aus Hydroxysäuren mit 6 bis 24 C-Atomen und ein- oder mehrwertigen Alkoholen mit Alkylenoxiden in Gegenwart eines Doppelmetallcyarudkatalysators, enthaltend eine Doppelmetallcyanidkomplexverbindung der allgemeinen Formel (I)

M¹ ₐ[M²(CN)_{b}(A)_{c}]_{d} · fM¹ _{g}Xₙ · h(H₂O) · eL, (I)

in der
- M¹: Zn²⁺,
- M²: mindestens ein Metallion, ausgewählt aus der Gruppe bestehend aus Fe³⁺und Co³⁺,
wobei M¹ und M² gleich oder verschieden sind,
- X: ein Anion, ausgewählt aus der Gruppe, bestehend aus Halogenid, Hydroxyd, Sulfat, Carbonat, Cyanid, Thiocyanat, Isocyanat, Cyanat, Carboxylat, Oxalat oder Nitrat,
und
- L: einen mit Wasser mischbaren Liganden, ausgewählt aus der Gruppe, bestehend aus Alkoholen, Aldehyden, Ketonen, Ethern, Polyethern, Estern, Harnstoffen, Amiden, Nitrilen und Sulfiden,
wobei a, b, c, d, g und n so ausgewählt sind, daß die Elektroneutralität der Verbindung gewährleistet ist,
- e: eine Zahl, die auch 0 sein kann,
- f: eine gebrochene oder ganze Zahl größer 0 oder 0 und
- h: eine gebrochene oder ganze Zahl größer 0 oder 0
bedeuten. Das erfindungsgemäße Verfahren ist dann dadurch gekennzeichnet, daß die Doppelmetallcyanidkomplexverbindung auf feste, inerte, ungeschäumte Träger aufgebracht oder in diese eingebracht ist oder zu Formkörpem geformt ist.

Die Erfindung beruht unter anderem auf der Beobachtung, daß im Falle aliphatischer Alkohole mit mehr als 5 C-Atomen die Alkoxylierungsreaktion in Gegenwart der erfindungsgemäß eingesetzten Katalysatoren schon nach einer sehr kurzen Induktionszeit anspringt. Die kurze Induktionszeit, die oft nur 15 Minuten und darunter beträgt, ist auch insofern überraschend, da im Falle der Alkoxylierung von niedermolekularen Polyolen mit vergleichbarem Molekulargewicht in Gegen-wart von Doppelmetallcyanidkomplexverbindungen die Reaktion erst nach vergleichsweise langen Induktionszeiten von 2 Stunden und mehr anspringt.

Die erfindungsgemäß eingesetzten Doppelmetallcyanidkatalysatoren enthalten eine Doppelmetallcyanidkomplexverbindung, die durch Umsetzung von Lösungen von Metallsalzen mit Lösungen von Alkali- oder Erdalkalimetallcyanometallaten oder Cyanometallatsäuren erhältlich sind. Zur entstandenden Fällungssuspension wird üblicherweise sofort nach dem Fällungsvorgang eine wassermischbare, ein oder mehrere Heteroatome enthaltende Komponente zugegeben. Die Komponente kann bereits in einer oder in beiden Eduktlösungen vorhanden sein.

Derartige Verfahren sind beispielsweise in US 3,278,457, US 3,278,458, US 3,278,459 und US 3,427,256 beschrieben.

Diese gliedern sich üblicherweise in folgende Verfahrensschritte:
a) Hinzufügen einer wäßrigen Lösung eines wasserlöslichen Metallsalzes der allgemeinen Formel (II)

   M¹ ₘ(X)ₙ (II),

   wobei M' und X die oben angegebene Bedeutung haben
   und m und n ganze Zahlen sind, die den Wertigkeiten von M¹ und X genügen, so daß die Elektroneutralität der Verbindung (II) gewährleistet ist,
   zu einer wäßrigen Lösung einer Cyanometallat-Verbindung der allgemeinen Formel (III)

   MaM²(CN)_{b}(A)_{c} (III),

   wobei
   M Wasserstoff oder ein Alkalimetall- oder ein Ammoniumion bedeutet,
   M² und A die oben angegebene Bedeutung haben, wobei A gleich mit oder verschieden von X sein kann, und a, b und c ganze Zahlen sind, die so ausgewählt sind, daß die Elektroneutralität der Cyanidverbindung gewährleistet ist,
   wobei eine oder beide Lösungen gegebenenfalls mindestens einen wassermischbaren, Heteroatome enthaltenden Liganden enthalten können, der ausgewählt ist aus der Gruppe, bestehend aus Alkoholen, Aldehyden, Ketonen, Ethern, Polyethern, Estern, Harnstoffen, Amiden, Nitrilen, Sulfiden oder wie in WO 98/16310 beschrieben, funktionalisierten Polymeren.
b) Vereinigen der in Schritt a) gebildeten wäßrigen Suspension mit einem wassermischbaren Heteroatome enthaltenden Liganden, ausgewählt aus der beschriebenen Gruppe, der gleich mit oder verschieden sein kann dem Liganden aus Schritt a), und
c) gegebenenfalls Abtrennen der Multimetallcyanidverbindung aus der Suspension.

Für das erfindungsgemäße Verfahren ist es vorteilhaft, als Cyanometallat-Verbindung die Säure zu verwenden, da es hierbei nicht zu einem Saizanfall kommt.

Diese einsetzbaren Cyanometallat-Wasserstoffsäuren sind in wäßriger Lösung stabil und gut handhabbar. Ihre Herstellung kann, wie in W. Klemm, W. Brandt, R. Hoppe, Z. Anorg. Allg. Chem. 308, 179 (1961) beschrieben, ausgehend vom Alkalicyanometallat über das Silbercyanometallat zur Cyanometallat-Wasserstoffsäure erfolgen. Eine weitere Möglichkeit besteht darin, ein Alkali- oder Erdalkalicyanometallat mittels eines sauren Ionenaustauschers in eine Cyanometallat-Wasserstoffsäure umzuwandeln, wie in F. Hein, H. Lilie, Z. Anorg. Allg. Chem. 270, 45 (1952), oder A. Ludi, H.U. Güdel, V. Dvorak, Helv. Chim. Acta 50, 2035 (1967) beschrieben. Weitere Möglichkeiten zur Synthese der Cyanometallat-Wasserstoffsäuren finden sich beispielsweise in "Handbuch der Präparativen Anorganischen Chemie", G. Bauer (Herausgeber), Ferdinand Enke Verlag, Stuttgart, 1981. Für eine technische Herstellung dieser Verbindungen, wie sie für das erfindungsgemäße Verfahren erforderlich ist, ist die Synthese über Ionenaustauscher der vorteilhafteste Weg. Die Cyanometallat-Wasserstoffsäure-Lösungen können nach der Synthese sofort weiterverarbeitet werden, es ist jedoch auch möglich, sie über einen längeren Zeitraum zu lagern. Eine solche Lagerung sollte unter Lichtausschluß erfolgen, um eine photokatalytische Zersetzung der Säure auszuschließen.

Der Anteil der Säure in der Lösung sollte größer 80 Gew.-%, bezogen auf die Gesamtmasse an Cyanometallat-Komplexen, vorzugsweise größer 90 Gew.-%, insbesondere größer 95 Gew.-%, sein.

Als Heteroatome enthaltende Liganden werden die oben beschriebenen organischen Substanzen verwendet.

Zur Herstellung des Doppelmetallcyanidkomplexes wird eine wäßrige Lösung einer Cyanometallat-Wasserstoffsäure oder eines Cyanometallat-Salzes mit der wäßrigen, Lösung eines Metallsalzes der allgemeinen Formel (II) vereinigt. Hierbei wird mit einem stöchiometrischen Überschuß des Metallsalzes gearbeitet. Vorzugsweise wird mit einem molaren Verhältnis des Metallions zur Cyanometallat-Komponente von 1,1 bis 7,0, bevorzugt 1,2 bis 5,0 und besonders bevorzugt von 1,3 bis 3,0 gearbeitet. Es ist vorteilhaft, die Lösung der Cyanometallat-Verbindung vorzulegen und die Metallsalzlösung zuzusetzen, es kann jedoch auch umgekehrt verfahren werden. Während und nach der Vereinigung der Eduktlösungen ist eine gute Durchmischung, beispielsweise durch Rühren, erforderlich.

Der Gehalt der Cyanometallat-Verbindung in der Lösung beträgt 0,1 bis 30 Gew.-%, bevorzugt 0,1 bis 20 Gew.-%, insbesondere 0,2 bis 10 Gew.-%, der Gehalt der Metallsalzkomponente in der Lösung beträgt 0,1 bis 50 Gew.-%, bevorzugt 0,2 bis 40 Gew.-%, insbesondere 0,5 bis 30 Gew.-%.

Die Heteroatome enthaltenden Liganden werden insbesondere nach der Vereinigung der beiden Eduktlösungen zu der entstehenden Suspension gegeben, wobei auch hier auf eine gute Durchmischung zu achten ist.

Es ist jedoch auch möglich, den Liganden bereits vollständig oder teilweise einer oder beiden Eduktlösungen zuzufügen. Dabei sollte man, aufgrund der Veränderung der Salzlöslichkeiten, den Liganden vorzugsweise der Cyanometallat-Verbindung-Lösung zusetzen.

Der Gehalt der Liganden in der Suspension sollte 1 bis 60 Gew.-%, vorzugsweise 5 bis 40 Gew.-%, insbesondere 10 bis 30 Gew.-% betragen.

Die erfmdungsgemäß verwendeten Multimetallcyanide können kristallin oder amorph sein. Unter kristallinen Multimetallcyaniden werden dabei Multimetallcyanide verstanden, deren stärkster Reflex im Röntgendiffraktogramm mindestens eine dreimal höhere Intensität hat als der Untergrund der Messung. Kristalline Multimetallcyanide können ferner kubisch sein oder Röntgendiffraktogramme zeigen, wie sie in EP-A 0 755 715 beschrieben sind. Unter amorphen Multimetallcyaniden sollen solche Multimetallcyanide verstanden werden, deren stärkster Reflex im Röntgendiffraktogramm eine Intensität kleiner dreimal der Intensität des Untergrundes aufweist oder die Röntgendiffraktogramme zeigen, wie sie in EP-A 0 654 302 und EP-A 0 743 093 beschrieben sind.

Das erfindungsgemäße Verfahren kann als diskontinuierliches (Batch-)Verfahren durchgeführt werden. Der Katalysator wird dabei im Starter, dem C₆-C₂₄-Alkohol, suspendiert. Nach erfolgter Umsetzung muß der Katalysator aus dem Endprodukt entfernt werden. Günstiger ist es, die Umsetzung mit einem geformten Katalysator durchzuführen, der nach der Umsetzung in einfacher Weise aus dem Endprodukt entfernt werden kann. Das erfindungsgemäße Verfahren wird mit einem Festbettkatalysator durchgeführt, da dann der Katalysator nach Beendigung der Reaktion in dem Festbett immobilisiert werden kann, so daß die Aufarbeitung des Fettallcoholalkoxylats vereinfacht wird. Die in der EP-A 0 659 798 beschriebenen eingeschäumten Doppelmetallcyanidkatalysatoren sind wegen ihrer unzureichenden mechanischen Festigkeit als Festbettkatalysatoren jedoch wenig geeignet. Daher sind bei dem erfindungsgemäßen Verfahren die Doppelmetallcyanidkomplexverbindungen auf einem festen, inerten, ungeschäumten Träger aufgebracht oder in diese eingebracht.

Die festen, ungeschäumten Träger sind makroskopische Formkörper, wie sie als Katalysatorträger üblich und bekannt sind, z.B. Stränge, Splitt, Tabletten, Netze, Packungen, Gewebe, Fasern, Kugeln sowie die Innenwände von Reaktoren. Die makroskopischen Formkörper können aus anorganischen und/oder organischen Materialien bestehen. Anorganische Materialien sind beispielsweise Oxide, Carbide, Nitride oder inerte Metalle. Beispiele für Carbide sind Übergangsmetallcarbide wie Wolframcarbid, sowie Siliziumcarbid oder Borcarbid. Als Nitride eignen sich beispielsweise Bornitrid; Siliciumnitrid oder Aluminiumnitrid. Als inerte Metalle gelten Metalle oder Metallegierungen, die sich im Reaktionsmedium der Doppelmetallcyanidsynthese sowie der Polyetheralkoholsynthese inert verhalten.

Beispiele hierfür sind Stähle, Aluminium, Edelmetalle, Nickel, Edelstähle, Titan, Tantal, Kanthal. Als Oxide kann man unter den genannten Reaktionsbedingungen inerte Metalloxide verwenden, insbesondere solche von Metallen der Gruppen IIA bis IVA sowie IB bis VIIIb, sowie oxidische Verbindungen, die Elemente der Gruppen IA bis VIIA und/oder der Metalle der Gruppen Ib bis VIIIb enthalten.

Die Herstellung der erfmdungsgemäßen Katalysatoren kann durch Aufbringen der Doppelmetallcyanidkomplexe auf die Oberfläche der geformten Träger oder durch Mischen von Doppelmetallcyanidkomplexen mit ungeformtem Trägermaterial und anschließende Formgebung erfolgen.

Für die Formgebung der Doppelmetallcyanidkatalysatoren stehen verschiedene Verfahren zur Verfügung.

Ein Verfahren, die Multimetallcyanidverbindung auf einen inerten Formkörper zu bringen, besteht im Aufsprühen einer Suspension des Multimetallcyanidpulvers in einer inerten Flüssigkeit. Als Sprühsuspension kann man entweder die Fällmaische der Multimetallcyanidverbindung verwenden, oder man suspendiert die bereits synthetisierte und möglicherweise getrocknete Multimetallcyanidverbindung in einer geeigneten Suspensionsflüssigkeit. Verwendet man direkt die Fällmaische zum Aufsprühen, so bevorzugt man Multimetallcyanidverbindungen, die unter Verwendung der Cyanometallat-Wasserstoff-Säure hergestellt wurden, da die als Nebenprodukte entstehenden Säuren während des Aufsprühvorganges von der Multimetallcyanidverbindung abgetrennt werden können. Verwendet man Alkaliund/oder Erdalkalicyanometallate als Ausgangskomponenten für die Fällung der Multimetallcyanidkatalysatoren, so entstehen als Nebenprodukte Alkali- und/oder Erdalkalisalze. Um diese von der Multimetallcyanidverbindung abzutrennen, kann man entweder die hergestellten Multimetallcyanidverbindungen aus der Fällsuspension abtrennen, nebenproduktfrei waschen, gegebenenfalls trocknen und dann erneut suspendieren, oder man sprüht die Fällsuspension direkt auf und versucht durch nachträgliches Waschen der geträgerten Multimetallcyanidverbindung die Alkali- und/oder Erdalkalisalze herauszuwaschen.

Um die Haftung der aufgesprühten Multimetallcyanidverbindung auf dem Formkörper zu erhöhen, kann man der Sprühsuspension zusätzlich ein organisches oder anorganisches Polymeres zusetzen. Dieses organische oder anorganische Polymere kann aktive Wasserstoffatome besitzen. Bevorzugt sind aber polymere Verbindungen, die keine aktiven Wasserstoffatome besitzen.

Ferner kann man zur Steigerung der Haftung reaktive, anorganische oder organische Stoffe entweder in Reinform, in Form von Lösungen, Dispersionen oder Emulsionen, einsetzen, die entweder thermisch oder photochemisch reagieren können, d.h. vernetzt werden und so einen starken Halt der Aktivmasse auf dem Träger ermöglichen.

Bevorzugt werden reaktive organische Polymere eingesetzt, deren Vemetzungsprodukte poröse Strukturen bilden.

Diese vorstehend beschriebenen Aufbringungsverfahren eignen sich auch, wenn man die Multimetallcyanidverbindungen auf die Innenwände von Reaktoren aufbringen will.

Neben dem Aufsprühen einer Multimetallcyanidverbindung enthaltenden Suspension kann man analog einem High-solids-coating-Verfahren direkt das Multimetallcyanidpulver auf den Formkörper aufbringen. In der Regel werden bei diesem Verfahren parallel zum Zulauf des Pulvers die Formkörper mit einer haftvermittelnden Flüssigkeit besprüht. Bei diesem Verfahren setzt man nur Multimetallcyanidpulver ein, die frei von jeglichen Nebenprodukten, wie Alkaliund/oder Erdalkalisalzen, sind. Wie auch beim Aufsprühen kann man entweder der haftvermittelnden Flüssigkeit oder dem Multimetallcyanidpulver organische oder anorganische Polymere zusetzen, die die Haftung zwischen Multimetallcyanidverbindung und Formkörper erhöhen.

Auch hier können reaktive, d.h. vemetzende, anorganische oder organische Komponenten zugesetzt werden.

Ein weiteres Verfahren, die Multimetallcyanidverbindung auf den Formkörper zu bringen, besteht in der direkten Synthese der Multimetallcyanidverbindung auf dem Formkörper. Dazu werden die verschiedenen Lösungen, die die Edukte, nämlich die Cyanometallat- und die Metallsalzkomponente enthalten, entweder zeitgleich oder in kurzem zeitlichen Versatz mit dem Formkörper in Kontakt gebracht. Das In-Kontakt-bringen des Formkörpers mit den Lösungen kann durch Besprühen, Tauchen, Tränken, Imprägnieren oder ähnlichen Prozeduren geschehen. Die organischen, mit Wasser mischbaren und Heteroatome enthaltenden Liganden können in diesem Fall entweder einer der beiden oder beiden Lösungen zugesetzt werden. Mischen sich die beiden Flüssigkeiten auf dem Formkörper, so kommt es zur Fällung der Muttimetallcyanidverbindung direkt auf dem Formkörper. Durch das direkte Aufwachsen der Multimetallcyanidkristalle auf dem Formkörper ist in der Regel eine sehr gute Haftung auf dem Formkörper gegeben. Sollte die Haftung der Multimetallcyanidkomponente dennoch ungenügend sein, kann in eine der beiden oder in beide Lösungen ein haftvermittelndes organisches oder anorganisches Polymeres zugesetzt werden. Verwendet man als Cyanometallatkomponente eine Cyanometallatwasserstoffsäure, so können die dabei entstehenden Säuren über die Gasphase abgezogen werden. Bei der Verwendung von Alkali- und/oder Erdalkalicyanometallaten verbleiben nach dem Aufsprühen Alkali- und/oder Erdalkalisalze als Nebenprodukte auf dem Katalysator. Diese Nebenprodukte können dann durch Waschen des Katalysators mit Wasser oder anderen, die Nebenprodukte lösenden Flüssigkeiten oder Flüssigkeitsgemischen entfernt werden. Auch dieses Verfahren ist geeignet, Innenwände von Reaktoren mit Multimetallcyanidverbindungen zu beschichten.

In den bisher beschriebenen Herstellverfahren für die erfindungsgemäßen Katalysatoren werden die Multimetallcyanidverbindungen auf inerte Formkörper aufgebracht.

Eine weitere Möglichkeit besteht darin, Multimetallcyanidverbindungen in einer festen Matrix einzuschließen. Die feste Matrix kann anorganischer oder organischer Natur sein.

Zum Einschluß eines Doppelmetallcyanidkomplexes in eine anorganische Matrix kann man die entsprechende Doppelmetallcyanid-Verbindung in Metallsäureester, bzw. Alkoxymetallaten suspendieren. Durch Zugabe von Base oder Säure lassen sich die Metallsäureester zu festen Stoffen polymerisieren. Bevorzugt sind dabei die Ester der Silizium-, Aluminium-, Titan- und/oder Zirkonsäure.

Als organische Komponenten lassen sich alle Stoffe bzw. Stoffgemische verwenden, in denen das Doppelmetallcyanid suspendiert werden kann und welche auf irgendeine Art und Weise zu Feststoffen polymerisiert werden können.

Die Polymerisation sollte so durchgeführt werden, daß die entstehenden Feststoffpartikel in einer Festbettanordnung verwendet werden können. Ferner sollten die erhaltenen Feststoffpartikel eine hinreichende Porosität besitzen, um den An- und Abtransport der Edukte bzw. Produkte zum Doppelmetallcyanid zu ermöglichen. Zur Verbesserung der Porosität ist es möglich, bei der Polymerisation Hilfsstoffe zuzusetzen, die durch physikalische oder chemische Behandlungen nach der Polymerisation wieder entfernt werden.

In einer weiteren bevorzugten Ausführungsform des erfindungsgemäßen Verfahrens erfolgt die Alkoxylierung in Gegenwart eines Doppelmetallcyanidkatalysators, der herstellbar ist durch Umsetzung eines Metallsalzes mit einer Cyanometallwasserstoffsäure, wobei mindestens eine der beiden wäßrigen Eduktlösungen, welche das Metallsalz beziehungsweise die Cyanometallwasserstoffsäure enthalten, einen gegenüber den Reaktanten der Katalysatorsynthese inerten anorganischen oder organischen unlöslichen Feststoff enthält. Solche Katalysatoren weisen eine hohe katalytische Aktivität auf.

Als anorganische Stoffe werden vorzugsweise Oxide, Nitride, Carbide, inerte Metalle oder Metallegierungen eingesetzt. Als Oxide können Oxide von Metallen der Gruppen IIB bis VIIIB und Oxide von Metallen der Gruppen IIIA und IVA sowie Mischungen daraus eingesetzt werden. Besonders bevorzugt werden Oxide von Metallen der Gruppe III A, IV A, IIIb, IVb bis VIb, Manganoxide, Eisenoxide, Cobaltoxide, Nickeloxide und Zinkoxid sowie Mischungen daraus verwendet. Als Nitride können Siliciumnitrid, Bornitrid, Vanadiumnitrid, Chromnitrid oder Wolframnitrid verwendet werden. Als Carbide werden bevorzugt die kovalenten oder metallischen Carbide verwendet, beispielsweise Siliciumcarbid, Borcarbid, Titan-, Zirkon-, Hafnium-, Vanadium-, Niob-, Tantal-, Molybdän- oder Wolframcarbid.

Die anorganischen Feststoffe werden entweder in Form von feinem Pulver oder als Splitt, Fasern, Fasergewebe oder Formkörper, wie Kugeln, Strängen oder Sternen, zugesetzt. Bevorzugt werden Pulver, Splitt sowie Fasern und Fasergewebe. Die BET-Oberflächen der Feststoffe liegen vorzugsweise zwischen 0.1 und 1000 m²/g, besonders bevorzugt zwischen 1 und 900 m²/g und insbesondere zwischen 10 und 800 m²/g.

Organische inerte Feststoffe können natürliche oder synthetische Polymere sein, aber auch Graphit, Ruß, Aktivkohlen sowie oxidativ behandelte Aktivkohlen. Natürliche Polymere sind beispielsweise Cellulose, Keratine und wasserunlösliche Pektine. Synthetische Polymere sind beispielsweise Polyethylen, Polypropylen, Polystyrol, Polyurethan, Polycarbonat, Polyacrylat, Polymethacrylat, Polyamid sowie Polyether oder Polyester. Bevorzugt sind Cellulose, Polyurethane und Polyalkylene. Die Zugabe erfolgt in Form von Pulvern, Fasern, Fasergeweben, Formkörpern, Granulaten.

Die inerten Feststoffe werden zumeist in der 0,05 bis 20-fachen Menge des zu fällenden Doppelmetallcyanids eingesetzt. Bevorzugt ist die 0,1- bis 10-fache Menge, besonders bevorzugt die 0,2- bis 5-fache Menge.

In einer bevorzugten Ausführungsform der Erfindung wird ein anorganischer oder insbesondere organischer Feststoff eingesetzt, der unter den Herstellungsbedingungen des Doppelmetallcyanidsalzes inert ist, sich jedoch unter den Reaktionsbedingungen bei der Herstellung des Fettalkoholalkoxylats löst und nach der Herstellung des Fettalkoholalkoxylats in diesem verbleibt, beispielsweise Polyester.

Besonders bevorzugt weisen die in dem erfindungsgemäßen Verfahren eingesetzten Doppelmetallcyanidkomplexverbindungen eines oder mehrere, insbesondere alle der nachstehenden Merkmale auf:
- L ist tert.-Butanol
- h oder e können gleich 0 sein.

Die oben beschriebenen Multimetallcyanidverbindungen eignen sich aufgrund ihrer hohen Aktivität hervorragend zur Synthese von Fettalkoholalkoxylaten. Die verwendeten Katalysatorkonzentrationen sind kleiner 1 Gew.-%, bevorzugt kleiner 0,5 Gew.-%, besonders bevorzugt kleiner 1000 ppm, insbesondere bevorzugt kleiner 500 ppm, speziell bevorzugt kleiner 100 ppm, bezogen auf die Gesamtmenge des hergestellten Fettalkoholalkoxylats. Die Herstellung des Fettalkoholalkoxylats kann sowohl kontinuierlich als auch diskontinuierlich erfolgen. Die Synthese kann in Suspensions-, Festbett-, Fließbett oder Schwebebettverfahren erfolgen. Die Temperaturen bei der Synthese liegen zwischen 50°C und 200°C, bevorzugt zwischen 90°C und 150°C.

Als Fettalkohole eignen sich alle Alkohole, die mehr als 6 C-Atome besitzen, wobei die Alkohole weitere, gegenüber Alkylenoxiden nicht reaktive funktionelle Gruppen, wie Estergruppen, aufweisen können. Bevorzugt sind Alkohole mit 6 bis 24 C-Atomen, besonders bevorzugt sind Alkohole mit 9 bis 20 C-Atomen. Diese Alkohole können gesättigt sein, wie Dodekanol oder Tridekanol, oder ungesättigt sein wie die Terpenalkohole Geraniol, Linalool oder Citronellol. In Frage kommen auch die Ester von gesättigten oder ungesättigten Hydroxysäuren mit 6 bis 24 C-Atomen, bevorzugt 6 bis 20 C-Atomen, mit ein- oder mehrwertigen Alkoholen, insbesondere Glycerin, wie das Glycerid der 12-Hydroxyoctadecensäure (z.B. aus Rizinusöl). Weitere Beispiele für Fettalkohole sind Hexanol, Heptanol, Oktanol, Dekanol, Undekanol, Tetradekanol, Pentadekanol, Hexadecanol, Heptadekanol, Oktadecanol, Nonadekanol, Hexenol, Heptenol, Oktenol, Nonenol, Dekenol und Undekenol.

Das eingesetzte Alkylenoxid ist vorzugsweise aus der Gruppe bestehend aus Ethylenoxid, Propylenoxid oder Butylenoxid oder deren Mischungen ausgewählt.

### Beispiele

### Herstellung der Doppelmetallcyanidkatalysatoren

### Vergleichsbeispiel 1

1000 ml stark-saurer Ionenaustauscher (K2431 der Bayer AG - Leverkusen) werden zweimal mit 180 g wäßriger HCl-Lösung (37 Gew.-%) regeneriert und anschließend mit Wasser solange gewaschen, bis der Ablauf neutral ist. Danach wird eine Lösung von 80,8 g K₃[Co(CN)₆] in 250 g Wasser auf die Austauschersäule gegeben. Es wird solange eluiert, bis der Auslauf wieder neutral ist. Das Co:K-Verhältnis im gewonnenen Eluat ist größer 10:1. 1305 g des Eluats werden auf 40°C temperiert und anschließend unter Rühren mit einer Lösung von 80,0 g Zn(II)-Acetat-Dihydrat in 240 g Wasser versetzt. Anschließend werden zur Suspension 276,4 g tert.-Butanol gegeben. Die Suspension wird bei 40°C weitere 30 min gerührt. Danach wird der Feststoff abgesaugt, auf dem Filter mit 300 ml tert.-Butanol gewaschen und bei Raumtemperatur getrocknet.

### Vergleichsbeispiel 2

400 ml stark-saurer Ionenaustauscher (K2431) werden zweimal mit 180 g wäßriger HCl-Lösung (37 Gew.-%) regeneriert und anschließend mit Wasser solange gewaschen, bis der Ablauf neutral ist. Danach wird eine Lösung von 40 g K₃[Co(CN)₆] in 120 g Wasser auf die Austauschersäule gegeben. Es wird solange eluiert, bis der Auslauf wieder neutral ist. Das Co:K-Verhältnis im gewonnenen Eluat ist größer 10:1. 397,5 g des Eluats (insgesamt 795 g) werden auf 40°C temperiert, mit 1 ml Lutensol TO 7 (BASF Aktiengesellschaft - Ludwigshafen, DE) versetzt und unter starkem Rühren (20.000 min⁻¹) mit einer Lösung von 20 g Zn(II)-Acetat-Dihydrat in 70 g Wasser versetzt. Zu der gebildeten Suspension werden 69,1 g tert.-Butanol gegeben. Die Suspension wird noch eine Minute lang stark gerührt, anschließend wird bei 40°C mit einem Magnetrührer noch 30 Minuten lang weitergerührt. Danach wird der Feststoff abgesaugt, auf dem Filter mit 300 ml tert.-Butanol gewaschen und bei 30°C im Vakuum (14 mbar) getrocknet.

### Vergleichsbeispiel 3

13 g K₃[Co(CN)₆] werden in 263,35 g Wasser gelöst, auf 40°C erwärmt und gut gerührt. Zu dieser Lösung werden unter Rühren 26,65 Zinkchlorid in 26,65 g Wasser und nach 15 Minuten 84 g Dimethoxyethan zugegeben. Die erhaltene Suspension wird noch 30 Minuten lang weitergerührt, der Feststoff abgesaugt und mit 300 ml Dimethoxyethan gewaschen und getrocknet.

### Herstellung von Fettalkoholpropoxylaten und Fettalkoholbutoxylaten

### Vergleichsbeispiele 4 bis 9

In einem 10 1-Kessel wird die in der Tabelle angegebene Menge Tridecanol N (OH-Zahl: 276,4 mg KOH/g) vorgelegt. Nach Zugabe der in der Tabelle angegebenen Menge des gemäß Vergleichsbeispiel 1 hergestellten Katalysators wird bei 105°C eine Stunde lang evakuiert (kleiner 1 mbar). Das Vakuum wird mit Stickstoff gebrochen, anschließend werden bei der in der Tabelle angegebenen Temperatur 150 g Propylenoxyd beziehungsweise Butylenoxyd zudosiert. Nach Anspringen der Reaktion, was am Druckabfall erkannt wird, wird der Rest der in der Tabelle angegebenen Menge Propylenoxyd beziehungsweise Butylenoxyd zudosiert. Nach Ende der Reaktion wird das Fettalkoholalkoxylat durch zwei Lagen eines Tiefenfilters K900 heiß filtriert. Die leichtflüchtigen Bestandteile werden durch Anlegen eines Vakuums entfernt und das Fettalkoholalkoxylat analysiert.

### Propoxylierung von Rizinusöl

### Vergleichsbeispiele 10 bis 12

In 100 g Rizinusöl (Glycerid der 12-Hydroxyoctadecensäure) wird bei 60°C die in der Tabelle angegebenen Menge des gemäß Beispiel 3 hergestellten Katalysators mit einem Turrax-Rührer 10 Minuten lang intensiv eingerührt. In einem 10 1-Reaktor wird bei Raumtemperatur der Hauptteil der in der Tabelle angegebenen Menge Rizinusöl vorgelegt und die Katalysatorsuspension hinzugegeben. Es wird evakuiert und langsam auf 110°C erwärmt. Es werden 200 g Propylenoxid zudosiert und das Anspringen der Reaktion abgewartet. Anschließend wird die restliche Menge Propylenoxid zudosiert Nach Ende der Reaktion wird 0,5 Stunden lang evakuiert und mit 2 Gew.-% Ambosol (Clariant AG, Frankfurt) und 2 Gew.-% Wasser aufgearbeitet.

### Herstellung von Fettalkoholethoxylat

### Vergleichsbeispiel 13

140 g Dodecanol N werden mit 0,13 g des gemäß Beispiel 2 hergestellten Katalysators (entpricht 150 ppm bezogen auf das Endprodukt) in einem Rührautoklaven unter Stickstoffatmosphäre gemischt. Der Kessel wird evakuiert und bei 105°C werden 720 g Ethylenoxid zudosiert. Der Ethylenoxiddruck beträgt zunächst 6 bar absolut. Das Anspringen der Reaktion nach ca. 15 Minuten wird am einsetzenden Druckabfall erkannt. Es wird noch ca. 2 Stunden lang Ethylenoxid so zudosiert, daß der Druck 7 bar absolut und die Temperatur 125°C nicht überschreiten. Nach weiteren 4 Stunden wird das erhaltene Produkt zweimal filtriert. Es wird ein Fettalkoholethoxylat mit folgenden analytischen Werten erhalten: OH-Zahl: 48,2 mg KOH/g; pH-Wert: 7,65; mittlere Molmasse: M_{w} ist gleich 1187 g/mol; Verteilung: D ist gleich 1,035.

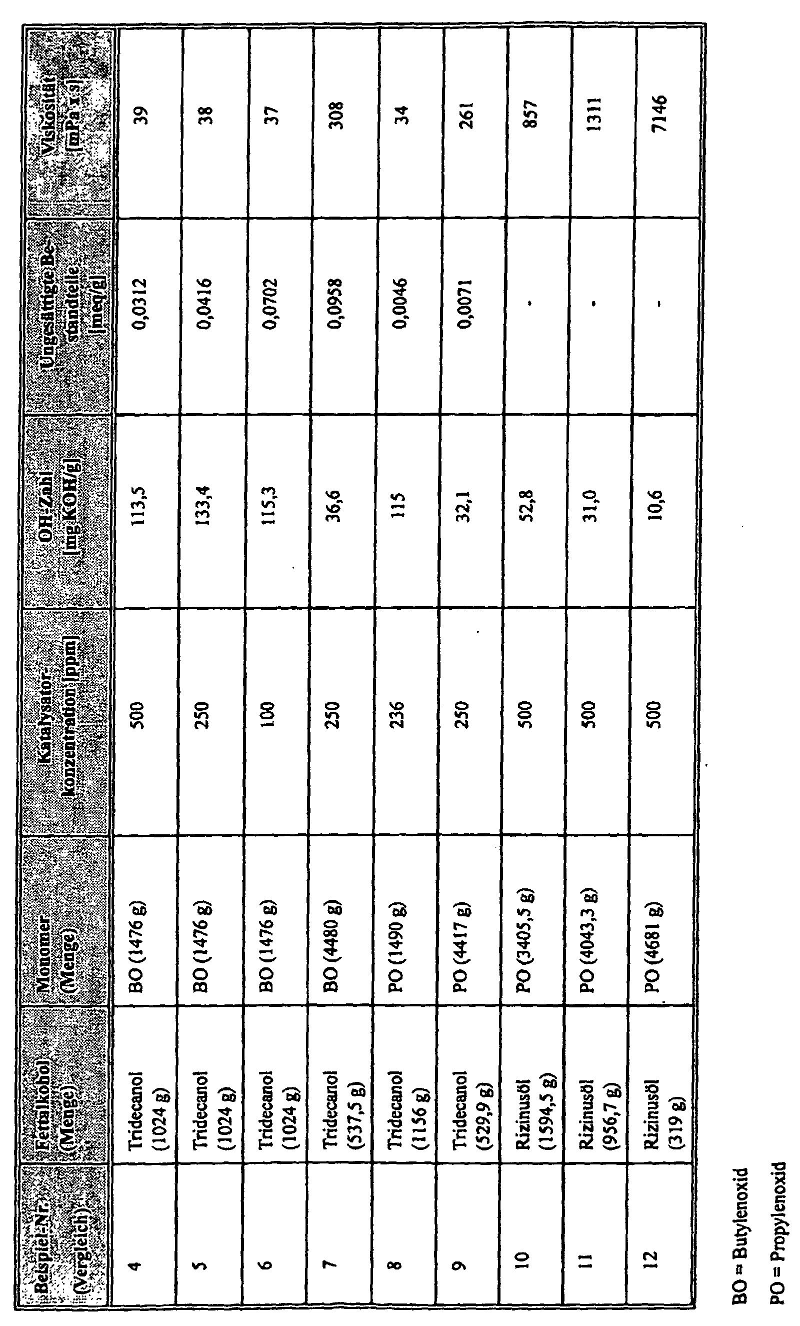

## Patentansprüche

1. Verfahren zur Herstellung von Fettalkoholalkoxylaten durch Alkoxylierung von einwertigen aliphatischen Alkoholen mit 6 bis 24 C-Atomen oder von Estern aus Hydroxysäuren mit 6 bis 24 C-Atomen und ein- oder mehrwertigen Alkoholen mit Alkylenoxiden in Gegenwart eines Doppelmetallcyanidkatalysators, enthaltend eine Doppelmetallcyanidicomplexverbindung der allgemeinen Formel (I),
M¹ ₐ[M²(CN)_{b}(A)_{c}]_{d} · fM¹ _{g}Xₙ · h (H₂O) · eL, (I)
in der
M¹ Zn²⁺
M² ein Metallion, ausgewählt aus der Gruppe bestehend aus Fe³⁺ und Co³⁺,
A Cyanid,
X ein Anion, ausgewählt aus der Gruppe, bestehend aus Halogenid, Hydroxyd, Sulfat, Carbonat, Cyanid, Thiocyanat, Isocyanat, Cyanat, Carboxylat, Oxalat oder Nitrat,
L einen mit Wasser mischbaren Liganden, ausgewählt aus der Gruppe bestehend aus Alkoholen, Aldehyden, Ketonen, Ethern, Polyethern, Estern, Harnstoffen, Amiden, Nitrilen und Sulfiden,
wobei a, b, c, d, g und n so ausgewählt sind, daß die Elektroneutralität der Verbindung gewährleistet ist,
e eine Zahl, die auch 0 sein kann,
f eine gebrochene oder ganze Zahl größer 0 oder 0 und
h eine gebrochene oder ganze Zahl größer 0 oder 0
bedeuten,
wobei die Doppelmetallcyanidkomplexverbindung herstellbar ist durch Umsetzung einer wäßrigen Lösung eines wasserlöslichen Metallsalzes der allgemeinen Formel (II)
M¹ ₘ(X)ₙ, (II)
mit einer wäßrigen Lösung einer Cyanometallat-Verbindung der allgemeinen Formel (III)
MₐM²(CN)_{b}(A)_{c}, (III)
worin M Wasserstoff, ein Alkalimetall- oder Ammoniumion bedeutet und im übrigen die Indices wie obenstehend definiert sind und darüber hinaus m so gewählt ist, daß die Elektroneutralität der Verbindung (II) gewährleistet ist, wobei eine oder beide wäßrige Eduktlösungen einen wassermischbaren organischen Liganden enthalten kann, gegebenenfalls Vereinigung der so erhaltenen wäßrigen Suspension mit einem wassermischbaren organischen Liganden, und Abtrennung der erhaltenen Doppelmetallcyanidkomplexverbindung aus der Suspension, **dadurch gekennzeichnet, daß** die Doppelmetallcyanidkomplexverbindung auf feste, inerte, ungeschäumte Träger aufgebracht oder in diese eingebracht ist.

2. Verfahren nach Anspruch 1, **dadurch gekennzeichnet, daß** bei der Herstellung des Doppelmetaltcyanidkatalysators die Umsetzung der wäßrigen Eduktlösungen in Gegenwart eines festen, inerten, ungeschäumten Trägers erfolgt oder die nach der Umsetzung erhaltene wäßrige Suspension, gegebenenfalls nach Vereinigung mit dem Liganden L, auf einen festen, inerten, ungeschäumten Träger aufgebracht und das Lösungsmittel entfernt wird.

3. Verfahren nach einem der Ansprüche 1 oder 2, **dadurch gekennzeichnet, daß** die Doppelmetallcyanidkomplexverbindung eines oder mehrere der nachstehenden Merkmale aufweist:
- L ist tert.-Butanol
- h oder e können gleich 0 sein.

4. Verfahren nach einem der Ansprüche 1 bis 3, **dadurch gekennzeichnet, daß** mit Ethylenoxid, Propylenoxid und/oder Butylenoxid alkoxyliert wird.

5. Verfahren nach einem der Ansprüche 1 bis 4, **dadurch gekennzeichnet, daß** Alkohole mit 9 bis 20 C-Atomen alkoxyliert werden.

6. Verfahren nach einem der Ansprüche 1 bis 5, **dadurch gekennzeichnet, daß** Glycerinester von Hydroxysäuren mit 6 bis 20 C-Atomen alkoxyliert' werden.

## Claims

1. A process for the preparation of fatty alcohol alkoxylates by alkoxylating monohydric aliphatic alcohols of 6 to 24 carbon atoms or esters of hydroxyacids of 6 to 24 carbon atoms and monohydric or polyhydric alcohols with alkylene oxides in the presence of a double metal cyanide catalyst, containing a double metal cyanide complex of the formula (I)
M¹ ₐ[M²(CN)_{b}(A)_{c}]_{d}.fM¹ _{g}Xₙ.h(H₂O).eL, (I)
where
M¹ is Zₙ²⁺,
M² is a metal ion selected from Fe³⁺ and Co³⁺,
A is cyanide,
X is an anion selected from the group consisting of halide, hydroxide, sulfate, carbonate, cyanide, thiocyanate, isocyanate, cyanate, carboxylate, oxalate and nitrate, and
L is a water-miscible ligand, selected from the group consisting of alcohols, aldehydes, ketones, ethers, polyethers, esters, ureas, amides, nitriles and sulfides,
a, b, c, d, g and n being selected so that the electroneutrality of the compound is maintained,
e is a number which can also be 0,
f is a fraction or integer greater than 0 or 0 and
h is a fraction or integer greater than 0 or 0,
in which the double metal cyanide complex can be prepared by reacting an aqueous solution of a water-soluble metal salt of the formula (II)
M¹ ₘ(X)ₙ (II)
with an aqueous solution of a cyanometallate compound of the formula (III)
MₐM²(CN)_{b}(A)_{c} (III)
where M is hydrogen or an alkali metal or ammonium ion and moreover the indices are as defined above and furthermore m is chosen so that the electroneutrality of the compound (II) is ensured, it being possible for one or both aqueous starting material solutions to contain a water-miscible organic ligand, if required combining the resulting aqueous suspension with a water-miscible organic ligand, and separating off the resulting double metal cyanide complex from the suspension, wherein the double metal cyanide complex is applied to solid, inert, unexpanded carriers or is introduced into said carriers.

2. A process as claimed in claim 1, wherein, in the preparation of the double metal cyanide catalysts, the reaction of the aqueous starting material solution is carried out in the presence of a solid, inert, unexpanded carrier or the aqueous suspension obtained after the reaction, if necessary after combination with the ligand L, is applied to solid, inert, unexpanded carrier and the solvent is removed.

3. A process as claimed in either of claims 1 and 2, wherein the double metal cyanide complex has one or more of the following features:
- L is tert-butanol
- h or e may be equal to 0.

4. A process as claimed in any of claims 1 to 3, wherein alkoxylation is effected with ethylene oxide, propylene oxide and/or butylene oxide.

5. A process as claimed in any of claims 1 to 4, wherein alcohols of 9 to 20 carbon atoms are alkoxylated.

6. A process as claimed in any of claims 1 to 5, wherein glyceryl esters of hydroxyacids of 6 to 20 carbon atoms are alkoxylated.

## Revendications

1. Procédé de production d'alcoxylates d'alcools gras au moyen d'une alcoxylation de monoalcools aliphatiques possédant 6 à 24 atomes de C ou d'esters d'hydroxyacides possédant 6 à 24 atomes de C et de monoalcools ou de polyols avec des oxydes d'alkylène en présence d'un catalyseur de cyanure de métal double (DMC) contenant un composé complexe de cyanure de métal double de formule générale (I),
M¹ ₐ[M²(CN)_{b}(A)_{c}]_{d}·fM¹ _{g}Xₙ·h(H₂O)·eL, (I)
dans laquelle
M¹ représente Zn²⁺
M² représente un ion métallique choisi dans le groupe formé par Fe³⁺ et Co³⁺,
A représente le cyanure
X représente un anion choisi dans le groupe formé par les ions halogénure, hydroxyde, sulfate, carbonate, cyanure, thiocyanate, isocyanate, cyanate, carboxylate, oxalate ou nitrate,
L représente un ligand miscible à l'eau, choisi dans le groupe formé par les alcools, les aldéhydes, les cétones, les éthers, les polyéthers, les esters, les urées, les amides, les nitriles et les sulfures,
a, b, c, d, g et n étant choisis de manière à garantir l'électroneutralité du composé,
e représente un nombre, pouvant être également égal à 0,
f représente un nombre fractionnaire ou un nombre entier supérieur ou égal à 0, et
h représente un nombre fractionnaire ou un nombre entier supérieur ou égal à 0,
le composé complexe de cyanure de métal double pouvant être obtenu au moyen de la réaction d'une solution aqueuse d'un sel métallique miscible à l'eau de formule générale (II)
M¹ ₘ(X)ₙ, (II)
avec une solution aqueuse d'un composé cyano-métallate de formule générale (III)
MₐM²(CN)_{b}(A)_{c}, (III)
dans laquelle M représente un atome d'hydrogène, un ion de métal alcalin ou d'ammonium et dans laquelle les indices sont par ailleurs tels que définis ci-dessus, et m étant en outre choisi de manière à garantir l'électroneutralité du composé (II), l'une ou les deux solutions aqueuses d'éduit pouvant contenir un ligand organique miscible à l'eau, éventuellement d'une combinaison des suspensions aqueuses ainsi obtenues avec un ligand organique miscible à l'eau, et d'une séparation du composé complexe de cyanure de métal double de la suspension, **caractérisé en ce que** le composé complexe de cyanure de métal double est déposé sur des supports solides, inertes, non expansés ou incorporé dans ces supports.

2. Procédé selon la revendication 1, **caractérisé en ce que**, lors de la préparation du catalyseur de cyanure de métal double, l'on réalise la réaction des solutions aqueuses d'éduit en présence d'un support solide, inerte non expansé ou **en ce que** l'on dépose la suspension aqueuse obtenue après la réaction, éventuellement après combinaison avec le ligand L, sur un support solide, inerte, non expansé et **en ce que** l'on élimine le solvant.

3. Procédé selon l'une quelconque des revendications 1 ou 2, **caractérisé en ce que** le composé complexe de cyanure de métal double présente l'une ou plusieurs des caractéristiques suivantes :
- L représente le tert-butanol
- h ou e peuvent être égaux à 0.

4. Procédé selon l'une quelconque des revendications 1 à 3, **caractérisé en ce que** l'on réalise l'alcoxylation avec de l'oxyde d'éthylène, de l'oxyde de propylène et/ou de l'oxyde de butylène.

5. Procédé selon l'une quelconque des revendications 1 à 4, **caractérisé en ce que** l'on réalise l'alcoxylation d'alcools possédant 9 à 20 atomes de C.

6. Procédé selon l'une quelconque des revendications 1 à 5, **caractérisé en ce que** l'on réalise l'alcoxylation d'esters glycériques d'hydroxyacides possédant 6 à 20 atomes de C.
